# EUROPEAN PATENT APPLICATION

(11) **EP 2 645 283 A2**
(43) Date of publication of application: **02.10.2013**
(21) Application number: 13161873.8
(22) Date of filing: 29.03.2013
(51) Int. Cl.: G06F 19/00

(54) **Method and apparatus for configuring and presenting first aid guide in portable terminal**

(30) Priority: 30.03.2012 KR 20120033000
(71) Applicant: Samsung Electronics Co., Ltd, Gyeonggi-do 443-742 (KR)
(72) Inventor: Jang, Yong-Sung, Gyeonggi-do 443-742 (KR); Moon, Sung-Whan, Gyeonggi-do 443-742 (KR); Jung, Han-Seong, Gyeonggi-do 443-742 (KR)
(74) Representative: Nederlandsch Octrooibureau

(57) **Abstract**

A method operable in a portable terminal is provided, which enables a first aid guide to be displayed even in a locked screen state, whereby instructions for an emergency illness are provided. A first aid icon may be displayed in the locked screen state, wherein selection of the icon is detected as a request to display a first aid guide. If a preset illness has been stored in the portable terminal in association with the portable terminal user, the first aid guide corresponds to the preset illness. An emergency contact and treatment instructions corresponding to the illness may be displayed in the first aid guide.

## Description

### BACKGROUND

### Technical Field

The present disclosure relates generally to portable terminals, and more particularly, to a method and apparatus for presenting a first aid guide in a portable terminal.

### Description of the Related Art

With recent advances in consumer electronics, computing and telecommunications, modern electronic devices tend to provide diverse functions. For example, portable terminals such as smart phones, and tablet PCs provide numerous functions, such as multimedia players, e-book readers, document editors, game consoles, etc.

Many portable terminals use a touch screen to provide the various functions. To prevent the touch screen from malfunctioning by physical contact of the user in an idle mode of the portable terminal, various screen lock functions have been introduced. For example, screen lock functions such as slide unlock or pattern unlock employ a preset slide or pattern input, giving an effect of preventing the touch screen from being unintentionally unlocked by the physical contact of the user. Further, password protection can be implemented to prevent unauthorized parties from unlocking the terminal and accessing the user's private information.

The foregoing screen lock function has merit of protecting the portable terminal from malfunctioning, but requires some time to unlock the screen, which works as an obstacle to quickly using the portable terminal. For example, if the user or a person nearby has an accident and thus needs to make an emergency call with his/her portable terminal, unlocking the screen or entering a password might be cumbersome. Plus, if the user is in a state of unconsciousness with the screen-locked portable terminal, a third party cannot use the user's portable terminal without knowing the password.

In this case, when the third party needs to use the user's portable terminal due to the user's emergency, the person is prevented from using it due to the password locked screen. The third party is then forced to use alternative means to communicate the emergency or take other remediable action, which may come too late.

### SUMMARY

The present disclosure provides a method and apparatus for quickly and conveniently providing a first aid function at a portable terminal in a lock screen state when an emergency occurs, thereby guiding a user (or a third party) of the portable terminal to give first aid to a patient (who may be the user or another third party) correctly and promptly.

A method operable in a portable terminal is disclosed, which enables a first aid guide to be displayed even in a locked screen state, whereby instructions for an emergency illness are provided. A first aid icon may be displayed in the locked screen state, where selection of the icon is detected as a request to display a first aid guide. If a preset illness has been stored in the portable terminal in association with the portable terminal user, the first aid guide corresponds to the preset illness. An emergency contact and treatment instructions corresponding to the illness may be displayed in the first aid guide.

In an embodiment, a method operable in a portable terminal includes receiving a request to present a first aid guide in a locked screen state. Responsive to the request, a first aid guide is presented which including entries for at least one illness and at least one first aid treatment instruction corresponding to the at least one illness.

The method may further include executing a configure mode for receiving entries for at least one preset illness associated with a user of the portable terminal, at least one emergency contact corresponding to the preset illness, and at least one treatment instruction corresponding to the preset illness.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram of a portable terminal, according to an embodiment of the present invention;
FIG. 2 is a flowchart of a method of configuring a first aid guide in a configure mode, in the portable terminal of FIG. 1, according to an embodiment of the present invention; FIGS. 3A-3D are screen examples illustrating operations in the method of FIG. 2;
FIG. 4 is a flowchart of a method of presenting a first aid guide in the portable terminal, according to an embodiment of the present invention;
FIGS. 5A-5D are screen examples illustrating operations in the method of FIG. 4; and
FIGS. 6A-6D are screen examples illustrating operations of presenting a first aid guide in the portable terminal, according to another embodiment of the present invention.

### DETAILED DESCRIPTION OF EXEMPLARY EMBODIMENTS

Reference will now be made in detail to embodiments, examples of which are illustrated in the accompanying drawings, wherein like reference numerals refer to like elements throughout.

As used in this document, including the Claims section, the words "a" or "an" mean one or more than one. The term "plurality" means two or more than two. The term "another" is defined as a second or more. The words "comprising", "including", "having" and the like are open ended. Reference herein to "one embodiment", "embodiments", "an embodiment" or similar term means that a particular feature, structure, or characteristic described in connection with the embodiment is included in at least one embodiment. The appearances of such phrases in various places throughout this disclosure are not necessarily all referring to the same embodiment. Furthermore, the particular features, structures, or characteristics may be combined in any suitable manner on one or more embodiments without limitation. The terms "may" or "can" are used herein to refer to at least an optional element, feature, function, characteristic, advantage, etc., of a described embodiment. Terms such as "substantially" or "generally" signify equality or an approximation with respect to a parameter. Herein, the terms "he", "she", "his" and "her" are not gender specific and refer to any user.

Herein, the term "portable terminal" and "terminal" each denote any portable, e.g., hand held, electronic device capable of transmitting and/or receiving an electromagnetic signal. Examples of a portable terminal include a conventional feature phone, a smart phone, a tablet PC, , a personal digital assistant (PDP), etc.. Portable terminals typically operate with an operating system such as Windows 8®, iOS®, Android®, or the like.

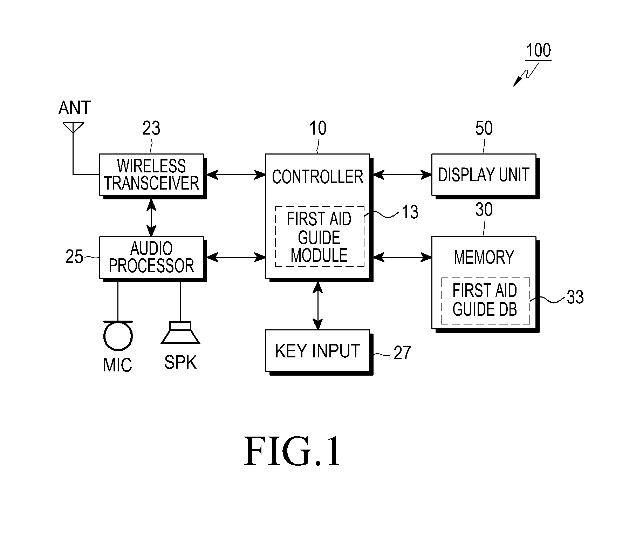

FIG. 1 is a functional block diagram of a portable terminal 100 in accordance with an exemplary embodiment. Portable terminal 100 may comprise a wireless transceiver 23 which includes a radio frequency (RF) unit and a modem. The RF unit includes an RF transmitter for up converting the frequency of a signal to be transmitted and amplifying the signal, and an RF receiver for low-noise amplifying a received signal and down converting the frequency of the received signal. The modem includes a transmitter for encoding and modulating the signal to be transmitted and a receiver for demodulating and decoding the signal received from the RF unit. A user may make a call or receive a call through the wireless transceiver 23 in a speaker phone or private mode.

An audio processor 25 may constitute a codec including a data codec and an audio codec. The data codec processes e.g., packet data, and the audio codec processes e.g., sound and multimedia files. The audio processor 25 performs a function of converting digital signals into analog signals and reproducing the analog signals with the audio codec, or converting analog signals input through a microphone to digital audio signals with the audio codec and transferring them to the modem. The audio codec may separately exist or may be incorporated into a controller 10.

A key input unit 27 may include keys for entering alphanumeric information and function keys for establishing various functions, or a touch pad. If a display unit 50 is implemented with a touch display screen using capacitive sensing or pressure sensing, the key input unit 27 may include only a predetermined minimum number of keys and the display unit 50 may replace some key functions.

A memory 30 may consist of a program memory and a data memory, and the program memory stores a program to control general operations of the portable terminal. The memory 30 may further include an external memory, such as compact flash (CF), secure digital (SD), micro secure digital (Micro-SD), mini secure digital (Mini-SD), extreme digital (xD), memory stick, and the like. The memory 30 may also include a disc, such as hard disc drive (HDD), solid state disc (SSD), and the like.

In embodiments of the present invention, the memory 30 includes a first aid guide database (DB) 33 which may include entries for illnesses, registration information of emergency contacts corresponding to the illnesses, and information regarding first aid treatments for the illnesses. The DB 33 may also include information regarding at least one preset illness (default illness) for the first aid guide. Here, a preset illness is one that has been specifically designated beforehand by a user of the portable terminal, and which may be associated with the owner of the terminal. If at least one preset illness has been stored, when the first aid guide is executed, then the default illness, an emergency contact corresponding to the default illness, and a first aid treatment for the default illness are preferably displayed automatically. Alternatively or additionally, the first aid guide DB 33 stored in memory 30 may be synchronized with a predetermined server through a wireless synchronization technology. The first aid guide DB 33 has various illnesses and corresponding first aid treatments, which may be presented in several representative languages (e.g., Korean, English, French, etc.).

The display unit 50 may be comprised of a liquid crystal display (LCD) or organic light emitting diodes (OLEDs, e.g., PMOLEDs or AMOLEDs), and outputs different display information generated in the portable terminal. The display unit 50 may operate as an input unit together with the key input unit 27 to control the portable terminal by including e.g., a capacitive or pressure sensitive touch screen.

In an embodiment of the present invention with respect to making an emergency call in the lock screen state, the display unit 50 provides a touch screen function, the touch screen being assumed to be locked. The lock screen may be unlocked according to a setting of the user.

The controller 10 includes at least one processor and controls general operations of the portable terminal, and may convert and control the operations of the portable terminal according to the user's input entered through the key input unit 27 or the display unit 50. Controller 10 may include a first aid guide module 13 which is a program that when executed, performs a method of configuring and providing a first aid guide in the portable terminal. The method generally involves receiving a request to present a first aid guide while the terminal is in a locked screen state. In response to the request, it is determined if a preset illness has been set via prior user input at the terminal 100. A first aid guide is displayed based on the determination. For instance, if a preset illness has been stored, the first aid guide displays information just associated with that illness, or, the preset illness information in a prioritized manner relative to other possible illnesses. Detailed description of methods performed by the controller 10 and first aid guide module 13 according to embodiments will be described below. In alternative implementations, first aid guide module 13 can be provided as a separate hardware or software module, separate from controller 10.

It will be appreciated that other devices, such as Bluetooth modules, camera modules, GPS modules, Wi-Fi modules, acceleration sensors, proximity sensors, magnetometer sensors, digital media broadcasting (DMB) receivers, etc., not currently shown in the block diagram of FIG. 1 may be included in the portable terminal and provide respective functions.

For example, a Wi-Fi module may replace the wireless receiver 23 for providing the function of receiving or making calls (or emergency calls) by using a voice over IP (VoIP) function. The Wi-Fi module may also replace a GPS module for use in determining location information of the portable terminal 100.

For example, if a request for displaying the first aid guide is made, the GPS module may be used to transmit information of a location where the request was made to an emergency contact corresponding to the illness in question. The GPS module may also be used to provide the information of the portable terminal's location for the emergency contact in a message format if a request for making a call to the emergency contact (e.g., in a speaker phone mode or a video call mode) failed, which will be described below in detail.

For example, there may be a plurality of camera modules which, in an embodiment of the present invention, may be driven to provide a function of the video call mode in the video call mode.

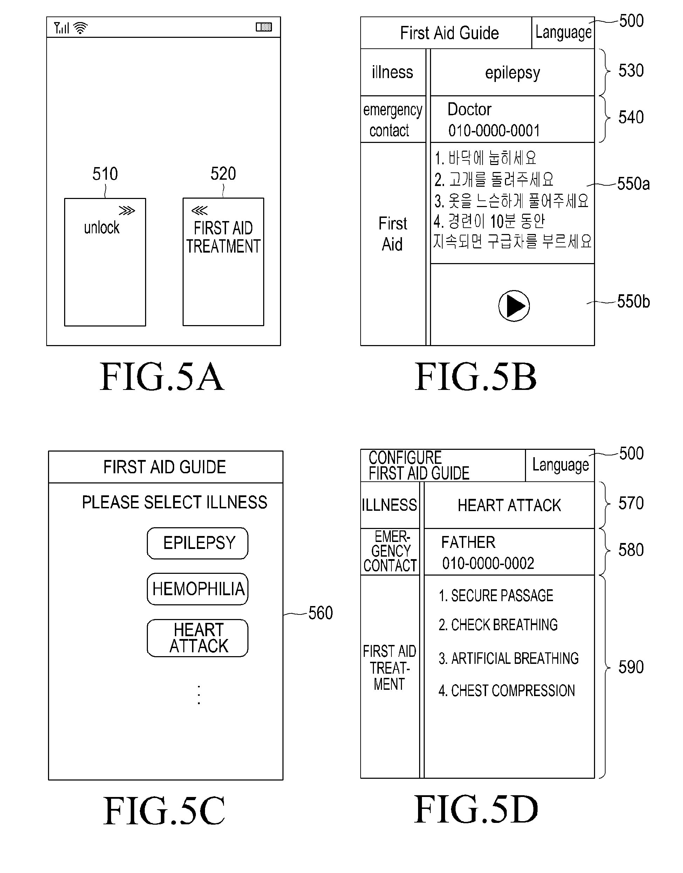

In embodiments of the invention, the first aid guide is accessed at the portable terminal 100 while its screen is locked, i.e., the screen is in a state rendering the portable terminal normally inaccessible unless a correct password is entered. A password can be, e.g., a number / letter sequence, touch pattern sequence and/or voice command. One way of enabling access to the first aid guide in the screen locked state is to display a first aid icon, as illustrated in FIG. 5A, icon 520. When the user selects the icon in a predetermined manner, such as a simple touch or a drag of the icon towards an unlock icon 510, the first aid guide is launched (automatically displayed). Alternatively or additionally, terminal 100 can be configured to recognize a predetermined voice command (e.g., the words "first aid" or "emergency" or "help") while in the screen locked state, which automatically launches the first aid guide.

In embodiments, at least one preset illness, emergency contact(s) and first aid treatment instructions associated with the illness are set up in a first aid guide configuration mode. For example, suppose the portable terminal owner (user) suffers from a chronic disease such as epilepsy. The user may configure the first aid guide so that in the event the user has an epileptic seizure and loses consciousness, a third party witnessing the event may check the user's portable terminal and launch the first aid guide via an icon displayed on the locked screen. With the first aid guide launched, the third party may use the information from the guide to deal with a corresponding first aid treatment for an epileptic seizure or call a registered contact (emergency contact) for the user.

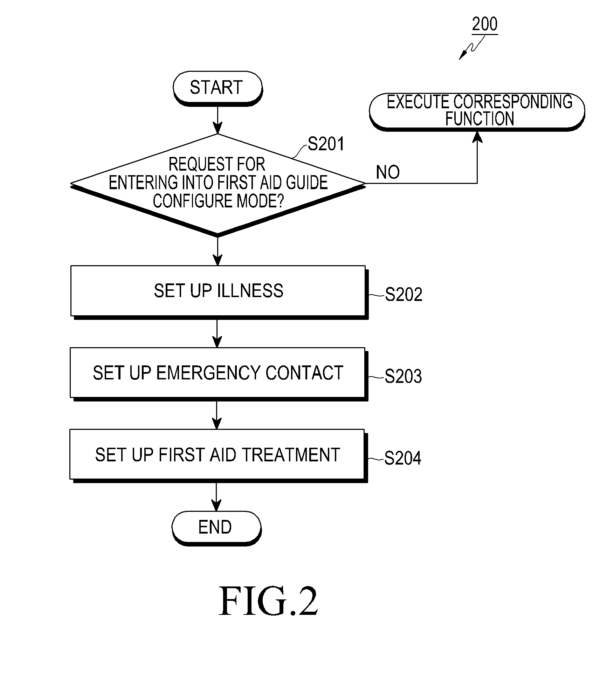

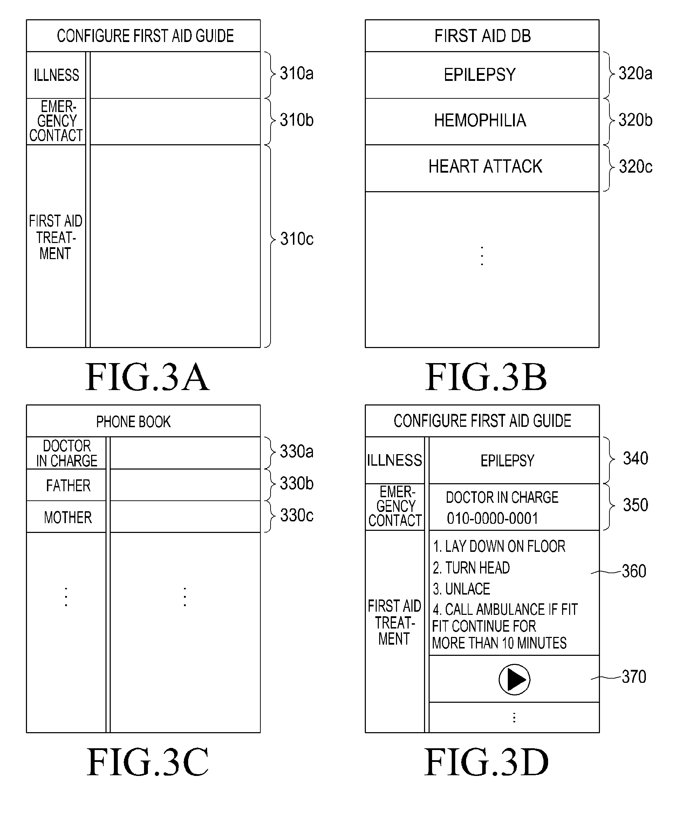

FIG. 2 is a flowchart of a method 200 of configuring a first aid guide in the portable terminal 100 in a configure mode according to an embodiment of the present invention. FIGS. 3A to 3D are screen examples to illustrate operations in the method of FIG. 2.The method is executed via controller 10 /first aid guide module 13.

In step S201, the user requests to enter into a first aid guide configure mode via a suitable menu entry in a settings mode or the like. The method sets up an execution environment of the first aid guide, and settings of the first aid guide are stored correspondingly to the execution environment set up by the user. Thus at step S202, the user sets up an illness by inputting text or selecting from a list of illnesses. The user further sets up an emergency contact (step S203) and first aid treatment advice (S204) to be displayed in the first aid guide executed in the lock screen state.

Referring to FIG. 3A, in configuring the first aid guide, the user may set up (designate) a preset illness 310a, an emergency contact 310b, and a first aid treatment 310c.

FIG. 3B is a screen example illustrating step S202 in which a preset illness is set up. The illness may be a name of disease the user of the portable terminal suffers from or injury that may possibly be caused from an accident, and may be written directly by the user or selected from a first aid DB as shown in FIG. 3B. The first aid DB, which is stored in the memory 30 (e.g., DB 33 of FIG. 1), may include information of illnesses, corresponding first aid treatments, hospital contacts, and the like. In the example of FIG. 3B, the first aid DB includes first aid treatments for epileptic seizure 320a, hemophilia 320b, heart attack 330c, etc.

Thus, if the user selects an illness using the first aid DB for configuring the first aid guide, the illness and a corresponding first aid treatment may be selected (established) automatically.

Upon completion of establishing the preset illness by the user's direct input or through a selection using the first aid DB, the user may set up an emergency contact (S203). The emergency contact may be a contact of a relative (e.g., parent) or of an expert in charge of the illness (e.g., a doctor in charge), which may be entered directly by the user or selected from a phone book as illustrated as 330a to 330c in FIG. 3C.

Upon completion of establishing the illness and the emergency contact in the first aid guide, the user may set up first aid treatment instructions (advice) corresponding to the illness. In embodiments, the first aid treatment advice may include text information for written instructions, still picture information for displaying first aid treatment in images, video information for displaying the first aid treatment in moving pictures, etc. Furthermore, in certain embodiments, the first aid treatment may include link information (e.g., URL information) enabling the foregoing text, still picture, or video information to be obtained by downloading or streaming.

An example of a first aid guide configured in the foregoing way in connection with FIGS. 3a-3c is illustrated in FIG. 3D.Here, epilepsy 340 is set up for the illness, and a name of a doctor in charge and the doctor's phone number 010-0000-0001 350 are set up for an emergency contact. In addition, text information 360 and video information 370 are set up for a first aid treatment.

In the case of the first aid guide configured as shown in FIG. 3D, when a user (or a third party) executes the first aid guide, he/she may call the emergency contact 350 for the epileptic emergency and find the first aid treatments 360 and 370.

Now, the first aid guide may be presented in various languages. For instance, the first aid guide may be presented in Korean as a default language but can be designed to take into account a situation in which a foreigner might use the first aid guide at the user's portable terminal when the user happens to lose his/her consciousness. In this regard, the user may set up the default language of the first aid guide (e.g., the illness, emergency contact, and first aid treatment), but the default language is preferably changeable at any time via selection of a suitable icon provided on the first aid guide screen.. To this end, the controller provides a corresponding translation function. The user may be provided with a list of prioritized languages to set up alternative language options, or, a sequence order of alternative languages can be established by default. It will be appreciated by an ordinary skilled person that the user may reconfigure the first aid guide (e.g., reestablish an illness, an emergency contact, a first aid treatment, a display language, or the like) through e.g., a menu at a later time.

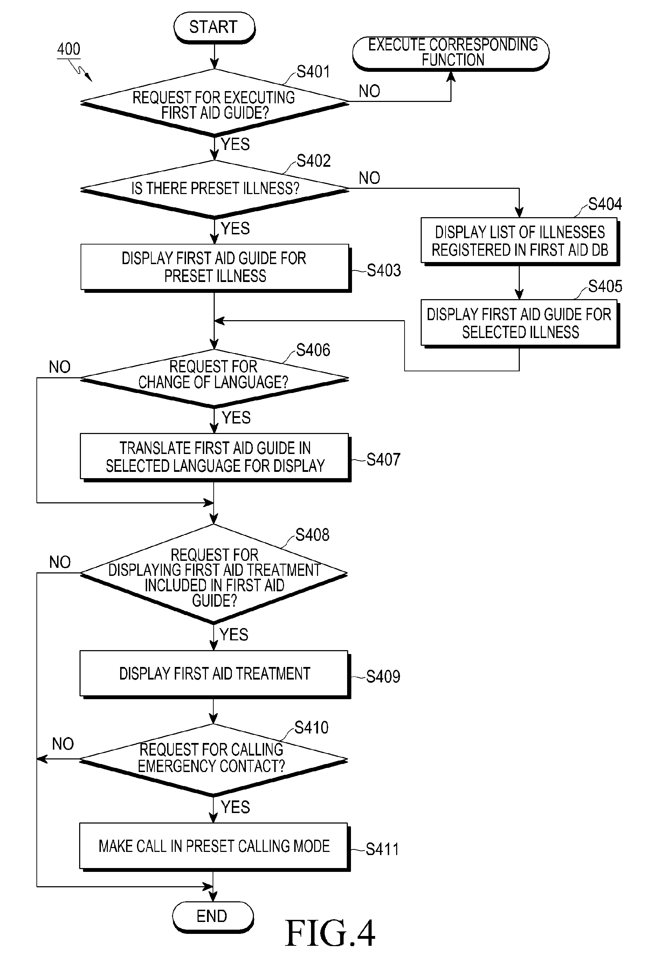

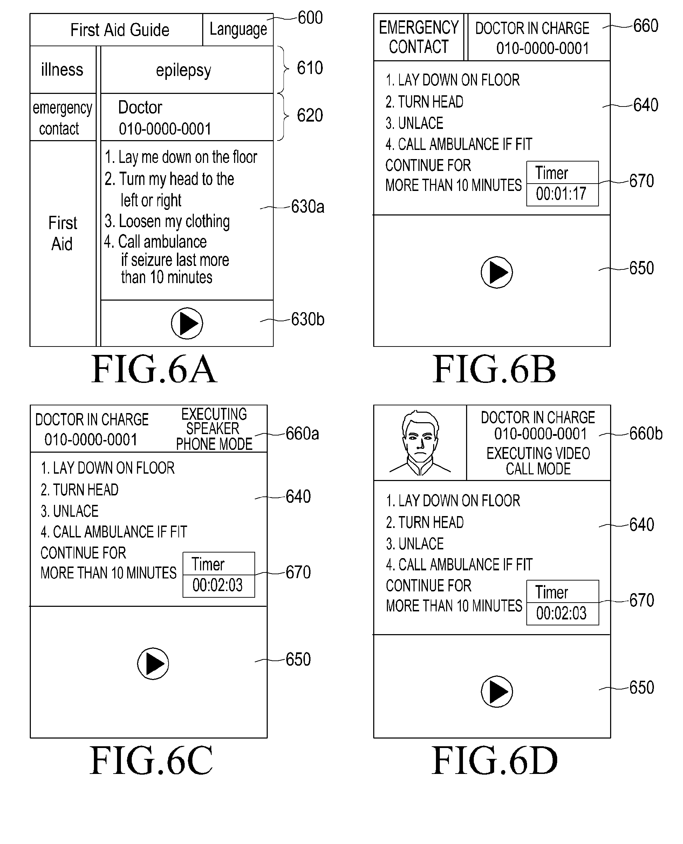

FIG. 4 is a flowchart of a method 400 of presenting a first aid guide in the portable terminal according to an embodiment of the present invention. FIGS. 5A-5D and 6A-6D are screen examples illustrating various operations in the method of FIG. 4 according to respective implementations.

At step S401, the terminal 100 detects whether a request is made to execute the first aid guide while the terminal is in a locked screen state. To this end, an example of a locked screen is shown in FIG. 5A. The locked screen includes an unlock icon 510 and a first aid treatment guide icon 520. When the user touches guide icon 520 in a predetermined manner, the first aid guide is launched. For instance, the system may be configured such that guide icon 520 is simply touched to launch (execute) the first aide guide, and/or the guide icon 520 may be slid into the unlock icon 510 by corresponding drag input to launch the first aid guide. (As described earlier, a portable terminal with a touch screen slides a screen unlock image 510 (e.g., to the right) responsive to a user's drag input, and then if a set password is entered the touch screen is unlocked. Although not shown in FIG. 5A, if the touch screen is locked in a pattern lock format, the user may release (i.e., unlock) the lock screen by entering a preset pattern.)

With the first aid guide executed, controller 10 determines if there is a preset illness (e.g., the default illness) by reading the first aid DB 33 stored in the memory 30. In an embodiment, if the user sets up a particular illness in advance, the first aid guide in connection with the particular illness is preferentially displayed. If a preset illness has been stored as described earlier, the first aid guide related to (corresponding to) the preset illness is displayed at S403. For example, if the preset illness to be displayed preferentially (or by default) is epilepsy" then as shown in the example of FIG. 5B, text for epilepsy 530 is displayed for the illness, a corresponding contact 540 for the emergency contact, and corresponding treatments 550a (e.g., text information) and 550b (e.g., moving picture information) for the first aid treatment. Note that text in FIG. 5B is displayed in Korean because it is assumed that Korean is the default language set up by the user. A language icon 500 can be selected to automatically change the displayed Korean text to another language under control of controller 10.

As described above in connection with FIGS. 2 and 3A-3D, one or more first aid treatments may be established and may be diversified into text, still image like a photo, moving picture, link information, etc., which may be added by the user or obtained from a service server.

Through the foregoing step S403, the user (or the third party) who requested for displaying the first aid guide may immediately see the first aid guide for the particular disease (e.g., epilepsy). For example, if an epileptic seizure occurs to the user of the portable terminal who suffers from the epilepsy, the third party passing by the user may request to display the first aid guide at the user's portable terminal, referring to the first aid guide to know how to deal with the user in the epileptic seizure.

In steps S404 and S405, determining that there is no preset illness, the controller 10 displays a list of illnesses registered in the first aid DB and controls display of the first aid guide for an illness selected by the user (or the third party), as illustrated in the example of FIG. 5C.

For instance, if the user having no chronic disease (i.e., no predetermined specific illness) happens to find a third party in an emergency at a particular location, the user may request to display the first aid guide at the user's portable terminal. At the user's request, an interface is presented, as in FIG. 5C, through which the user (or the third party) may select an illness he/she wants to identify by referring to the first aid DB 33 stored in the memory 30.

Assuming that a heart attack icon of FIG. 5C is selected for the illness through the interface, a first aid guide corresponding to the selected illness is displayed as shown in FIG. 5D. Here, it may be seen that the first aid guide including "heart attack" 570 selected by the user, an emergency contact 580 corresponding to the heart attack, and first aid treatment advice 590 corresponding to the heart attack is presented. In this case, the user (or the third party) may make a call to the emergency contact or request to display the first aid treatment.

In a case of the emergency contact represented by 540 in (b) of FIG. 5 and 580 in (d) of FIG. 5, a name of the contact (e.g., a doctor in charge) and a contact number (e.g., 010-0000-0002) are presented. Considering the user's privacy, the name of the emergency contact may not be necessarily disclosed. Thus, in an embodiment, in displaying the emergency contact in the first aid guide, the name of the contact may be alternatively represented as a temporary alias, or only a part of the contact may be represented, or the contact number may be replaced by a virtual number.

In steps S406 and S407, if requested by the user to change the language of the first aid guide, the controller 10 controls translation of the first aid guide information and audio accompanying video 550b, if any, and displays the first aid guide in a selected language, in response to the request. In this case, the user (or the third person) may request to translate a particular language (e.g., Korean) currently being used for displaying the first aid guide into another language (e.g., English) for display.

In steps S408 and S409, if a request is detected to display the first aid treatment included (displayed) in the displayed first aid guide, e.g., detecting selection of play icon in field 550b, the first aid treatment instructions are displayed as a still image or video in response to the request.

In embodiments, the first aid treatment may be text, still image, moving picture information, or the like, and, as discussed above, may also be link information enabling to find the text, still image, moving picture information, or the like. Thus, if requested by the user to display the first aid treatment, the controller 10 controls display of the first aid treatment stored in the memory 30 or obtained from a particular server (not shown).

As mentioned above, there may be a plurality of first aid treatments requested to be displayed by the user, which may be displayed one at a time, or simultaneously.

For example, assuming that the first aid treatments represented only in the text information and the video information are set up, either one of them may be displayed, as illustrated in FIG. 6A. Alternatively, as shown in FIG. 6B, both the text information 640 and the moving picture information 650 may be displayed in split-screens. That is, the screen may be split correspondingly to the number of the plurality of types or items of the information included in the first aid treatment, and the plurality of information items or types may be displayed simultaneously on the split-screen. For instance, if the user selects the video icon field 630a in FIG. 6A, the video may be played in the larger field 650 of FIG. 6B. Naturally, as the screen set-up changes, other information fields can be configured to disappear or change in size. For instance, as represented by 660 of FIG. 6B, the emergency contact field may be moved higher and still accessible for automatic calling, while other information fields (600, 610) are removed.

In the embodiments described above, the first aid guide for the preset illness is displayed following a selection of the icon 520 in FIG. 5A. In an alternative implementation, treatment advice such as those of fields 640, 650 are displayed on the lock screen instead of the icon 520. This enables a third party to simply glance at the portable terminal and obtain the treatment advice immediately, without the need to select an icon as 520. In this implementation, the size and location of the unlock icon 510 may be altered to make room for treatment advice fields.

In steps 5410 and S411, if a request is received to make a call to the emergency contact, the controller 10 controls performance of a predetermined calling mode with the emergency contact. The predetermined calling mode refers to a calling mode that may be performed without interfering with the display of the first aid treatment while the first aid treatment is displayed, and may include, not exclusively, a speaker phone mode, a video call mode, and the like. The predetermined calling mode may be performed while displaying the first aid treatment, or may be performed while pausing the display of the first aid treatment. Furthermore, the predetermined calling mode may be set up as the speaker phone mode or the video call mode by the user or third party, or may be selected by the third party or user from among the speaker phone mode and the video call mode when there is a request for making a call to the emergency contact.

If the predetermined calling mode involves a voice call in the speaker phone mode, the controller 10 controls to make the voice call to the emergency contact in the speaker phone mode at the request for making a call to the emergency contact. For example, as shown in FIG. 6C, the controller 10 may continue to display the first aid treatments 640 and 650 while proceeding with the speaker phone mode with the emergency contact. In this way, the user or third party may get advice about the first aid treatment through the ongoing voice call in the speaker phone mode while giving first aid to a patient (the user or a third party) by referring to the first aid treatment.

In addition, if the predetermined calling mode is the video call mode, the controller 10 controls making a video call with the emergency contact by driving a camera module (not shown) at the request for calling the emergency contact.

In this way, the user or third party may provide a current condition of the patient to the emergency contact or get advice about the first aid treatment from the emergency contact in video images while viewing the displayed first aid treatment.

In an embodiment, in the speaker phone or video call mode, an automatic recording function may be performed to automatically store contents of the calls made to the emergency contact. The recorded audio of the calls may be stored in the memory 30 under control of controller 10.

Again, in steps S410 and S411, the speaker phone mode and the video call mode are processed in screens obtained by splitting one of the former split-screens (e.g., the split-screens for displaying the first aid treatments), which was split before the speaker phone mode and the video call mode. Thus, as described above, the user may exchange opinions with the counterpart (a contact, such as the emergency contact) by performing the speaker phone mode or the video call mode, while referring to the first aid treatment being displayed in the display unit 50, and thus may efficiently give first aid to the patient.

In an embodiment variation, as represented by 670 in FIGS. 6B and 6D, a time elapsed since the display of the first aid treatment is displayed as a timer. With the timer, the user or third party may efficiently give first aid required, which may involve checking the time. For example, as the first aid treatment advice (having e.g., text information) represented by 640 in FIG. 6D, if it is instructed to call an ambulance if a fit continues for more than 10 minutes, the user is able to give first aid while conveniently checking the time elapsed with the timer 670. The timer 670 window may be displayed adjacent to the text of the time-dependent treatment instruction, as illustrated.

According to embodiments of the present invention thus described, a function of quickly and conveniently using a first aid guide is provided when an emergency occurs in the lock screen state of the portable terminal, thus allowing the user / owner (or a third party user) of the portable terminal to quickly and correctly give first aid to a patient (the user or another third party).

In a simpler alternative embodiment, no preset illness option is provided. Thus, in FIG. 4, for example, steps S402 and S403 could be skipped in this embodiment, wherein step S401 would be followed by step S404.

The above-described methods according to the present invention can be implemented in hardware, firmware or as software or computer code that can be stored in a recording medium such as a CD ROM, an RAM, a floppy disk, a hard disk, or a magneto-optical disk or computer code downloaded over a network originally stored on a remote recording medium or a non-transitory machine readable medium and to be stored on a local recording medium, so that the methods described herein can be rendered in such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein. In addition, it would be recognized that when a general purpose computer accesses code for implementing the processing shown herein, the execution of the code transforms the general purpose computer into a special purpose computer for executing the processing shown herein.

While the exemplary embodiments described herein have been particularly shown and described with reference to exemplary embodiments thereof, it will be understood by those of ordinary skill in the art that various changes in form and details may be made therein without departing from the spirit and scope of the invention as defined by the following claims and their equivalents.

## Claims

1. A method (400) operable in a portable terminal(100), the method comprising:
receiving (S401) a request to present a first aid guide while the terminal is in a locked screen state;
responsive to the request, determining (S402) if a preset illness is stored in memory via prior user input at the terminal; and
displaying (S403, S404) a first aid guide based on the determination.

2. The method of claim 1, wherein the displaying of the first aid guide comprises displaying (S403) the first aid guide for the preset illness if it is determined that the preset illness is stored.

3. The method of claim 1, wherein the displaying of the first aid guide comprises:
displaying a list of illnesses (S404) registered in a first aid database if it is determined that there is no preset illness stored; and
displaying (S405) the first aid guide for an illness selected from the displayed list.

4. The method of claim 1, further comprising translating (S407) the displayed first aid guide in a particular language for display when there a request for the translation is detected.

5. The method of claim 1, wherein the first aid guide comprises an illness, an emergency contact corresponding to the illness, and one or more first aid treatments corresponding to the illness.

6. The method of claim 5, further comprising displaying a time elapsed (670) since the display of the first aid guide while displaying the first aid guide.

7. The method of claim 5, wherein the first aid treatment comprises link information for presenting at least one of text information, still image information, and moving picture information.

8. The method of claim 5, wherein the displaying of the first aid treatment comprises
splitting a screen (640, 650) correspondingly to a number of the text information, the still image information, or the moving picture information, and displaying the text information, the still image information, and the moving picture information in the split-screens, respectively.

9. The method of claim 5, further comprising performing a preset calling mode (S411) when a request for making a call to the emergency contact is detected.

10. The method of claim 8, wherein the performing of the preset calling mode comprises
when there is a request for making a call to the emergency contact, proceeding to a video call mode and making a video call, the video call mode being processed in a screen obtained by further spitting one of plural split screens.

11. The method of claim 1, further comprising:
displaying a first aid guide (520) icon in the locked screen state;
wherein receiving a request to present a first aid guide comprises detecting a user selection of the first aid icon.

12. A method operable in a portable terminal, the method comprising:
receiving a request for configuring the first aid guide; and
configuring the first aid guide by setting up an illness, one or more emergency contacts corresponding to the illness, and one or more first aid treatments corresponding to the illness,
wherein the configured first aid guide is displayed when the portable terminal is in a lock screen state.

13. The method of claim 12, wherein the first aid treatment comprises at least one of text information, still image information, and moving picture information, and
link information for presenting at least one of the text information, the still image information, and the moving picture information.

14. The method of claim 13, wherein the first aid treatment is displayed in a screen split correspondingly to a number of the text information, the still image information, or the moving picture information.

15. An apparatus (10, 30, 50) of a portable terminal (100), the apparatus comprising:
a memory (30) that stores a first aid guide database (33);
a display unit (50) having a touch screen; and
a controller (10) configured to: i) receive a request (S401) to present a first aid guide database while the terminal is in a locked screen state; ii) responsive to the request, determine (S402) if a preset illness is stored in the memory via prior user input at the terminal; and iii) control display of a first aid guide (S403, S404) based on the determination.
